(19) Europäisches Patentamt
European Patent Office
Office européen des brevets

(11) Publication number: 0 365 044
A2

# (12) EUROPEAN PATENT APPLICATION

(21) Application number: **89121379.5**

(22) Date of filing: **24.07.85**

(51) Int. Cl.[5]: **A61K 37/02 , //C07K7/06**

(30) Priority: **02.08.84 GB 8419716**
**02.08.84 GB 8419715**

(43) Date of publication of application:
**25.04.90 Bulletin 90/17**

(60) Publication number of the earlier application in accordance with Art.76 EPC: **0 170 623**

(84) Designated Contracting States:
**AT BE CH DE FR GB IT LI LU NL SE**

(71) Applicant: **SANDOZ AG**
**Lichtstrasse 35**
**CH-4002 Basel(CH)**
(84) **BE CH FR GB IT LI LU NL SE**

Applicant: **SANDOZ-PATENT-GMBH**
**Humboldtstrasse 3**
**D-7850 Lörrach(DE)**
(84) **DE**

Applicant: **SANDOZ-ERFINDUNGEN**
**Verwaltungsgesellschaft m.b.H.**
**Brunner Strasse 59**
**A-1235 Wien(AT)**
(84) **AT**

(72) Inventor: **Borel, Jean-François**
**Dornacherweg 4**
**CH-4144 Arlesheim(CH)**
Inventor: **Donatsch, Peter**
**Herrenweg 34**
**CH-4123 Allschwil(CH)**
Inventor: **Hiestand, Peter**
**Schönenbuchstrasse 13A**
**CH-4123 Allschwil(CH)**
Inventor: **Ryffel, Bernhard**
**Schweizergasse 8**
**CH-4054 Basle(CH)**

## (54) Novel pharmaceutical use of (NVA)2-cyclosporine.

(57) Novel pharmaceutical use for the compound $(Nva)^2$-Cyclosporine, in particular use in the treatment of autoimmune diseases selected from the group consisting of:
c) Juvenile diabetes type I, d) Myasthenia gravis, a) Multiple sclerosis, b) Systemic lupus erythematosus, e) Haemolytic anaemia, and f) Glomerulonephritis.

EP 0 365 044 A2

# NOVEL PHARMACEUTICAL USE OF $(NVA)^2$-CYCLOSPORINE

The present invention relates to a new use, in particular a new pharmaceutical use, for the compound cyclosporin G, also known in accordance with current nomenclature and referred to throughout the present specification and claims as $(Nva)^2$-Cyclosporine, especially to its use in the preparation of pharmaceutical compositions for applcation in relation to said use.

$(Nva)^2$-Cyclosporine, which has the formula I

-A-B-Sar-MeLeu-Val-MeLeu-Ala-(D)Ala-MeLeu-MeLeu-MeVal- (I)

wherein A represents the residue of formula II

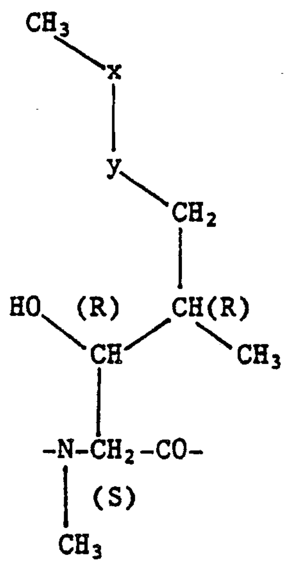

(II)

in which -x-y- is CH=CH-(trans), and B is Nva, is known and described together with processes for production as well as various pharmaceutical utilities, e.g. in US patent no. 4,288,431. Chemically $(Nva)^2$-Cyclosporine belongs to the distinct and now substantial class of natural and synthetic or semi-synthetic undecapeptides collectively designated as the cyclosporins [see for example US patent No. 4,117,118 (cyclosporin A or Cyclosporine); US patents Nos. 4,108,985 and 4,210,581 (cyclosporin C or $(Thr)^2$-Cyclosporine, and derivatives); as well as European patent publication No. 0,058,134 B1 and Helv. Chim. Acta. 65, Fasc. 5, pp. 1655 - 1677 (1982) disclosing yet further naturally occurring and synthetic or semi-synthetic cyclosporins including inter al. $(Leu)^{10}$-Cyclosporine, $[(D)Ser]^8$-Cyclosporine and $(Val)^{11}$-Cyclosporine].

Of the cyclosporins the parent compound, cyclosporin A or Cyclosporine, has so far received the most attention. As disclosed in the aforementioned US patent No. 4,117,118 it possesses anti-inflammatory and anti-arthritic properties, as well as immunosuppressive properties which render the compound of especial utility in the prevention of rejection following organ transplant operations as well as in the treatment of auto-immune diseases.

The primary area of clinical investigation of Cyclosporine has been in its application to recipients or organ, e.g. heart, lung, combined heart-lung, liver, kidney, pancreatic, bone-marrow, skin and corneal transplants and, in particular, allogenic organ transplants. Cyclosporine is now commercially available for use in relation to organ transplant and a measure of its remarkable success is provided by the uncommonly wide attention the compound has received, not only in the scientific literature, but also in the lay press world-wide. At the same time, investigation of the applicability of Cyclosporine to the treatment of various auto-immune diseases has been intensive and reports of results in-vitro, in animal models and in clinical trials are wide-spread in the literature. The variety of auto-immune diseases is in fact very large. Though

inevitably reported success for Cyclosporine has tended to vary in degree, e.g. from one auto-immune disease to another, broad utility is established and in some instances available results are sufficiently encouraging to warrant on-going world-wide specialist attention.

Specific auto-immune diseases for which Cyclosporine treatment has been investigated or proposed include multiple sclerosis, Guillain-Barré syndrome, uveitis, myesthenia gravis, Heymann nephritis, Grave's disease, Hashimoto's thyroiditis, juvenile diabetes type I, systemic lupus erythematodes, aplastic anaemia, pure red cell anaemia, idiopathic thrombocyctopaenia, polychondritis, sclerodoma, Wegner granulamatosis, dermatomyositis, chronic active hepatitis, autoimmune male infertility, psoriasis and psoriatic arthritis, Steven-Johnson syndrome, idiopathic sprue, Chron's disease, sarcoidosis, glomerulonephritis, interstititial lung fibrosis and primary billiary cirrhosis.

As disclosed e.g. in US patent specification No. 4,288,431, $(Nva)^2$-Cyclosporine possesses anti-inflammatory and anti-arthritic properties as well as immunosuppressive properties in test methods previously applied to Cyclosporine and to have the same utility profile and clinical utility as Cyclosporine. $(Nva)^2$-Cyclosporine like Cyclosporine may thus in its turn be anticipated to find application primarily in the prevention of transplant rejection as well as in the treatment of auto-immune disease. The present invention resides in the finding that, while $(Nva)^2$-Cyclosporine may be confirmed to possess the same general utility as Cyclosporine, i.e. in terms of its applicability to prevention or organ transplant rejection and to the treatment of auto-immune diseases as a broad class, its particular profile of activity, especially its particular profile in relation to immunosuppressive effect, render the compound surprisingly and unexpectedly advantageous in its application to certain specific diseases within the broad auto-immune disease group, namely:

c) Juvenile diabetes type I, d) Myasthenia gravis, a) Multiple sclerosis, b) Systemic lupus erythematosus; e) Haemolytic anaemia, and f) Glomerulonephritis.

More especially it may be shown that in relation to the above specified auto-immune diseases $(Nva)^2$-Cyclosporine exhibits particular and unexpected advantage in terms of e.g. level of activity and/or reduction or absence of undesirable side reactions in comparison with previously proposed therapy, e.g. employing Cyclosporine.

The particular and advantageous utility of $(Nva)^2$-Cyclosporine in the treatment of the above specified auto-immune diseases can be demonstrated in recognised standard animal models as well as in clinical trials conducted for example as follows:

## 1. JUVENILE DIABETES TYPE I

### BB/Worcester Rat Model

The trial is based on the general methodology described by Like et al. in Am.J.Pathol. 117, 92-97 (1984). For purposes of the test groups of BB/W (♂ and ♀) rats (a strain spontaneously developing auto-immune diabetes of the juvenile diabetes type I) aged 60 days are employed. Test groups receive test substance, i.e. dihydro-$(Val)^2$-Cyclosporine or $(Nva)^2$-Cyclosporine, at a dosage of from 5 to 25 mg/kg/day p.o. for 10 days. Control groups receive olive oil only in place of test substance. All test animals are screened regularly for the occurrence glycosuria.

On adiminstration of $(Nva)^2$-Cyclosporine at the above indicated dosage rates,marked reduction in the number of rats exhibiting diabetes, e.g. exhibiting abnormally elevated blood glucose levels/glycosuria is observed compared with controls.

## 2. MYASTHENIA GRAVIS

### Experimental Allergic Myasthenia Gravis (EAMG)

Testing is carried out in accordance with the general methodology described by V.A. Lennon et al. in J. Exp. Med. 141, 1365 - 1375 (1975). EAMG is induced in groups of 8 to 12 Lewis rats (♀) weighing ca. 150 - 200 g by intracutaneous injection of a mixture containing 1 part Freund's complete adjuvant (Difco, 0638-60) and 1 part of a solution comprising 10 µg purified acetylcholine receptor (obtained from Torpedo california rats). Additional adjuvant (pertussis, $1x10^{10}$ units) is administered s.c. into the hind paw pad. Autoantibody

titres are determined at weekly intervals using ELISA technique.

2.1 Prophylactic activity:

On administration of $(Nva)^2$-Cyclosporine at a dosage of from 25 to 50 mg/kg/day, p.o. 5 days a week commencing on the day of immunisation, inhibition of antibody formation compared with controls receiving placebo is observed.

2.2 Therapeutic activity:

On administration of $(Nva)^2$-Cyclosporine at dosages of from 25 to 50 mg/kg/day p.o. 5 days a week and commencing after autoantibodies have already been formed (generally ca. 14 days after immunisation) significant reduction in autoantibody titre is observed compared with control groups receiving placebo.

3. MULTIPLE SCLEROSIS

3.1 Preventive Activity in Experimental Allergic Encephalomyelitis (EAE)

Testing is carried out in accordance with the general methodology described by Borel et al. in Agents and Action, 6, 468 (1976). EAE is induced in groups of 8 to 12 Wistar (♀) or Lewis (♀) rats each weighing 150 to 200 g by intradermal injection into each hind foot pad of 0.1 ml of an emulsion comprising 2.5 g bovine spinal cord (lyophylised and reconstituted with 12 ml $H_2O$), 1.5 ml Arlacel A, 8.0 ml Nujol and 0.2 ml saline containing 20 mg killed, dried Mycobacterium phlei. $(Nva)^2$-Cyclosporine is administered at a dosage of from 25 to 50 mg/kg/day p.o. 5 days a week, commencing on the day of sensitisation and continuing for 3 weeks. Onset of EAE in control groups receiving no medication generally commences between 9 and 16 days after sensitisation and is marked by symptoms of paralysis in the hind limbs and tail. Test animals are examined daily for the symptoms of the disease and disease occurrence is scored as positive when complete involvement of both hind legs and the tail is observed. The test animals are kept under observation for a total period of 25 days.

On administration of $(Nva)^2$-Cyclosporine at the above indicated dosage rates substantial reduction of occurrence of EAE is observed over the test period in comparison with occurrence in control groups receiving placebo.

3.2 Activity in Established EAE

Testing is carried out analogously to 3.1, but with administration of $(Nva)^2$-Cyclosporine commencing on day 8 to day 9 after sensitisation (i.e. immediately prior to appearance of disease symptoms), at a dosage of from 25 to 50 mg/kg p.o. administered daily or every 2nd day, and continuing for ca. 14 days. During the treatment period animals are examined daily for symptoms of the disease, and scored as under 3.1.

On administration of $(Nva)^2$-Cyclosporine at the above indicated dosage rates substantial reduction of appearance of EAE disease symptoms is observed over the test period in comparison with appearance in control groups receiving placebo.

4. SYSTEMIC LUPUS ERYTHEMATOSUS

(NZB/NZW)F1 mouse model:

Trials are based on the (NZB/NZW)F1 mouse strain as described and discussed by Steinberg et al. in Bulletin on the Rheumatic Diseases 28, nos. 4-5, 940 - 946 (1977-78) published by The Arthritis Foundation, Atlanta, Georgia. Females of this strain spontaneously develop characteristica of the SLE syndrome including formation of anti-DNA and anti-erythrocytes autoantibodies as well as proteinurea at age ca. 5 to

7.5 months. The condition ultimately leads to death.

For the purpose of the trial groups of 6 to 8 ♀ mice are employed. Treatment with $(Nva)^2$-Cyclosporine at dosages of from 50 to 100 mg/kg/day p.o. administered 5x weekly and continuing for ca. 8 to 10 weeks commences i) prior to spontaneous development of autoantibodies, e.g. at ca. 5 months' age and ii) subsequent to spontaneous development of autoantibodies, e.g. at ca. 8 - 9 months of age. Anti-DNA and anti-erythrocyte antibody titres are measured at regular intervals during the trial period employing ELISA technique and during the trial period commencing from ca. 1 week prior to $(Nva)^2$-Cyclosporine administration. Additional parameters subject to control are development of proteinuria (measured 1x/week) and life span. Results in groups treated as under (i) and (ii) above indicate prophylactic and therapeutic effectiveness respectively.

On administration of $(Nva)^2$-Cyclosporine at the above indicated dosage levels, substantial reduction of autoantibody titres and occurrence of proteinuria as well as an increase in average life span is observed in both prophylactic and therapeutic treatment regimens as compared with results for control groups receiving placebo.

## 5. HAEMOLYTIC ANAEMIA

### Rat Erythrocyte Induced Anti-Mouse Erythrocyte Autoantibody Model

Testing is carried out in accordance with the general methodology described by Naysmith et al. in Immunological Rev. 55, 54 - 86 (1981). Four injections each comprising ca. $10^8$ well-washed rat erythrocytes are administered to groups of young, normal/healthy mice at days 0, 7, 14 and 21 of the test, injection being effected i.p.. From day 21 on the animals are bled at fixed 5 to 7 day intervals into citrate saline, and a direct Coomb's test is performed using a broad spectrum sheep anti-mouse immunoglobulin antiserum. Positive reaction in the Coomb's test is generally observed in control animals receiving no medication from ca. week 3 to 4 of the trial on. The trial is continued for ca. 10 to 12 weeks.

### 5.1 Prophylactic activity

On administration of $(Nva)^2$-Cyclosporine at a dosage of from 50 to 100 mg/kg/day p.o. administered 5 days a week commencing on day 0 of the trial and continuing for ca. 4 to 6 weeks, marked reduction in the number of animals reacting postively in the Coomb's test up until trial completion is observed compared with control groups receiving olive oil only.

### 5.2 Therapeutic activity

On administration of $(Nva)^2$-Cyclosporine at dosages of from 50 to 100 mg/kg/day, p.o. 5 days a week commencing after autoantibodies have already been formed (positive reaction in Coomb's test, generally ca. 21 to 28 days after day 0) and continuing for 4 to 6 weeks, significant reduction of response in the Coomb's test is observed as compared with control groups receiving olive oil only.

## 6. GLOMERULONEPHRITIS

### Back-Ground

Female NZB/W hybrid mice spontaneously develop renal disease characterised by immunoglobulin (Ig) and complement (C′) deposition at 3 to 6 months of age with histological glomerulonephritis and proteinurea from about 6 months and thus provide an appropriate animal model for the disease glomerulonephritis as it occurs in man.

### Method

Female NZB/W mice are randomly sorted into control and treatment groups of 10 mice each. Each mouse is ear-marked to permit individual identification. Experiments are started when the mice are aged 12, 24 or 36 weeks old and treatment continues for 12 weeks. $(Nva)^2$-Cyclosporine is administered at dosages of from 50 to 100 mg/kg given orally by gavage five times a week.

The degree of proteinurea is estimated by staining 10 µl urine spots on filter paper with bromophenol blue. A thin-layer-chromatography scanner connected to an intergrating computer is used to quantify the intensity of the stained urine spots compared to a serial dilution of bovine serum albumin. Levels of protein above 100 mg% are considered abnormal and positive.

Mice are sacrificed at the end of each experiment and the kidneys routinely prepared for histological examination. Direct immunofluorescent studies are performed using antisera directed against mouse Ig and C3 (Nordic). Glomerulonephritis is classified into (i) mild endothelialmesangial lesions, (ii) more severe segmental proliferative, and (iii) most severe membrano-proliferative type. Scoring is from no lesion progressing to severe lesion. Immunofluorescent scoring ranges from nothing to strong. Results are compared with a control group receiving placebo (olive oil) only. If an animal dies while having proteinurea during the experiment, it is considered positive.

On administration of $(Nva)^2$-Cyclosporine at dosages indicated above marked reduction of deposition of immunoreactants and histological evidence of glomerulonephritis is observed as compared with control groups receiving placebo only.

As previously indicated effectiveness of $(Nva)^2$-Cyclosporine in accordance with the present invention may also be demonstrated in clinical trials, for example in the case of treatment of Juvenile diabetes type 1, conducted as follows:

The trial is carried out employing volunteer, insulin-dependent diabetics, diagnosed as exhibiting Juvenile Diabetes Type I. Diagnosis is made on clinical groups in non-obese subjects with confirmed hyperlycaemia [National Diabetes Data Group, Diabetes 28, 1039 (1979)]. All subjects have a serum immuno-reactive C-peptide concentration within the normal fasting range, tested in the fasting state with and without glucagon stimulation (0.2 p mole/ml), and have received insulin therapy for less than 12 months.

Subjects are admitted to hospital for an average period of ca. 5 days at the beginning of the trial. Baseline creatinine clearance, urinalysis, serum creatinine, blood urea nitrogen, serum glutamic-oxaloacetic transaminase (SGOT), and alkaline phosphatase levels are determined to ensure that these are normal. Oral administration of test substance, i.e. $(Nva)^2$-Cyclosporine is commenced at dosages of from ca. 5 to 25 mg/kg/day/patient generally administered once or in divided dosages each 12 hours. Daily serum concentrations are measured by radioimmunoassay and individual dosage rates adjusted to maintain blood concentrations of from ca. 200 to 2,500 ng/ml at 12 hours. After discharge patients are seen weekly for 2 weeks and monthly thereafter. At each in-clinic visit blood is drawn for determination of test substance levels, creatinine and electrolyte concentrations and for hematological and liver function tests; basal and glucagon-stimulated plasma C-peptide concentrations are measured at 1 month and at 3 month intervals thereafter.

During the course of the trial all patients are treated with purified pork insulin (Iletin II) and are encouraged to effect administration 2x daily and to monitor blood glucose concentrations using visual matching or reference meter methods with reagent strips. They are instructed in a diabetic diet appropriate for maintainance of normal body weight and activity. Insulin dosage is adjusted as far as possible to achieve a mean blood glucose level of 7.8 m mole/litre before the main meals and evening snack. Insulin dosage is reduced when control of glycaemia is consistant with these targets or in order to avoid hypoglycaemia. Subjects for which insulin is completely withdrawn for at least 1 week without loss of target control or development or ketonuria and who need not resume insulin treatment during the course of the study are classed as no longer-insulin-requiring (NIR). Where subjects becoming NIR subsequently develop hyperglycaemia exceeding treatment goals, application of the oral hypoglycemic agent glybenclamide is undertaken.

Results indicate a marked and significant increase of NIR remission in subjects participating in the trial as compared with remissions (i.e. spontaneous NIR remissions) recorded for groups of juvenile diabetics for whom no therapy is attempted or in comparison with NIR remissions recorded in groups of juvenile diabetics receiving alternative intervention therapy. Furthermore results would indicate that therapy is markedly well tolerated, e.g. as evidenced by measurements for other physiological parameters measured during the course of the trial.

In accordance with the foregoing the present invention provides: the use of $(Nva)^2$-Cyclosporine for the manufacture of a pharmaceutical composition for the treatment of an autoimmune disease selected from the group consisting of: a) Juvenile diabetes type I, b) Myasthenia gravis, c) Multiple sclerosis, d) Systemic lupus erythematosus, e) Haemolytic anaemia, and f) Glomerulonephritis.

By "treatment" as used in the above definitions is to be understood both curative treatment as well as prophylactic treatment where appropriate, e.g. as indicated in the above described animal model test methods.

Doses for clinical use in accordance with the method of the invention will of course vary depending upon, e.g. the mode of administration, the particular autoimmune disease or the specific condition of the subject to be treated and the effect desired. In addition dosaging will generally require adjustment for individual patients receiving treatment in order to establish an appropriate long-term drug serum concentration, e.g. by administration of an initial daily starting or "loading" dose with subsequent dose adjustment (generally reduction) in accordance with achieved serum levels as determined, e.g. by regular RIA monitoring. However in general satisfactory results are obtained on administration of $(Nva)^2$-Cyclosporine in a dose range of from about 3 to about 30, e.g. from about 10 to about 25 mg/kg body weight/day administered to the patient orally once or, in divided doses, 2 or 3x a day. Where i.v. administration is indicated, for example in the initial phase of treatment, lower dosages, e.g. of the order from about 1 to about 10, e.g. ca. 3 to 5 mg/kg/day are generally indicated. A suitable oral daily dosage, e.g. for adult patients, is accordingly of the order of from about 225 to about 2,000, e.g. from about 750 to about 1,800 mg/day, and unit dosage forms for oral administration suitably comprise from about 75 to about 2,000, e.g. from about 250 to about 1,500 mg $(Nva)^2$-Cyclosporine/dose, together with a pharmaceutically acceptable diluent or carrier therefor.

$(Nva)^2$-Cyclosporine is tolerated at dosages contemplated for use in accordance with the present invention. Indeed it has very surprisingly been found that while $(Nva)^2$-Cyclosporine generally possesses a high order of immunosuppressive activity, and may be shown to possess particularly advantageous properties in relation to immunosuppressive use in accordance with the present invention, e.g. as hereinbefore indicated, it is unexpectedly lacking in side effects, in particular hepato- an nephro-toxic effects, hitherto experienced employing Cyclosporine as medication. This relative freedom from undesirable side effects may for example be demonstrated in standard short-term toxicity trials.

One such trial is carried out employing 3 groups of 5 HAN Wistar rats. Group 1 receives 100 mg/kg $(Nva)^2$-Cyclosporine/day, Group 2 receives 100 mg/kg $(Nva)^2$-Cyclosporine/day and Group 3 (control) remains untreated. Both $(Nva)^2$-Cyclosporine and Cyclosporine are administered in solution in olive oil by means of a stomach tube. The following parameters are evaluated: body weight; mortality; serum and urine analysis; kidney and liver analysis, macroscopy and histology (urine volume, kidney weight, creatine clearance, Na and K levels, SGPT and SGOT levels etc); an neurological symptoms. Results obtained for neurological symptoms, mortality, kidney biochemistry and hystology (in particular swelling/regeneration of tubular epithelial cells and tubular necrosis) and liver biochemistry in Group 1 are in all cases directly comparable with results obtained for control Group 3 and show over all marked superiority in comparison with results obtained for Group 2. No adverse reaction in Group 1 as compared with Group 2 is observed in respect of other parameters measured.

Suitable galenic formulations for the administration of cyclosporins, including the current commercially available Cyclosporine drink solution, are described and claimed in DOS 29 07 460 (= Japanese patent application No. 27228/1979, U.K. patent specification No. 2 015 339 B and US patent No. 4,388,307). Pharmaceutical compositions for use in accordance with the present invention may be prepared directly analogously to the methods disclosed therein, employing $(Nva)^2$-Cyclosporine as active ingredient. Liquid oral compositions comprising both $(Nva)^2$-Cyclosporine (e.g. drink solutions), as the Cyclosporine drink solution itself, are suitably administered together with a chocolate flavouring agent, e.g. as described in Example 1 of the aforementioned US patent No. 4,388,307.

The following are examples for the preparation of liquid pharmaceutical preparations suitable for the administration of $(Nva)^2$-Cyclosporine. All percentages are by weight.

| EXAMPLE 1: DIHYDRO $(VAL)^2$-CYCLOSPORINE DRINK SOLUTION | |
|---|---|
| COMPONENT | CONTENT |
| i) Dihydro-$(Val)^2$-Cyclosporine<br>ii) Ethanol (absolute)<br>iii) Cremophor® RH 40<br>iv) Maisine®<br>v) Labrafil® 2125 | 5 - 10 % , e.g. 7.0 %<br>10 - 12 % , e.g. 10.5 %<br>ca. 4 % , e.g. 2.8 %<br>30 - 40 % , e.g. 65.0 %<br>to 100 % total |
| [Cremopher® RH 40 = reaction product of hydrogenated castor oil and ethylene oxide in a molar ratio of ca. 1:40 - available from BASF AG, Ludwigshafen DT. Maisine® = trans-esterified maize oil -available from ETS. Gattefossé, Boulogne-Bri lancourt FR. Labrafil® 2125 = polyoxyethylated kernel oil - available from ETS. Gattefossé, Boulogne-Brillancourt FR.] | |

The desired quantity of i) is dissolved in components ii) through iv) in conventional manner, the solution brought to an end-volume of 100 % by addition of v) and the resultant mixture filled into a small vial. For the purposes of administration, the solution is conveniently mixed into a flavour-masking composition, e.g. into chocolate-flavoured milk, prior to administration.

| EXAMPLE 2: $(NVA)^2$-CYCLOSPORINE DRINK SOLUTION | |
|---|---|
| COMPONENT | CONTENT mg/ml |
| i) $(Nva)^2$-Cyclosporine<br>ii) Ethanol (absolute)<br>iii) Labrafil® 2125<br>iv) Corn oil to a total of | 100.0<br>150.0<br>350.0<br>922.0 (≡ 1 ml) |

The individual components are compounded as in example 1 and the obtained solution filled into individual containers in an amount giving the required dosage of i). The solution is preferably ingested undiluted.

## Claims

1. The use of $(Nva)^2$-Cyclosporine for the manufacture of a pharmaceutical composition for the treatment of an autoimmune disease selected from the group consisting of:
a) Multiple sclerosis, b) Systemic lupus erythematosus, c) Juvenile diabetes type I, d) Myasthenia gravis, e) Haemolytic anaemia, and f) Glomerulonephritis.